(19) Europäisches Patentamt / European Patent Office / Office européen des brevets

(11) **EP 1 870 650 A1**

(12) **EUROPEAN PATENT APPLICATION**

(43) Date of publication:
26.12.2007 Bulletin 2007/52

(51) Int Cl.:
*F26B 5/06* (2006.01)     *A61K 9/19* (2006.01)

(21) Application number: 07110675.1

(22) Date of filing: 20.06.2007

| | |
|---|---|
| (84) Designated Contracting States:<br>AT BE BG CH CY CZ DE DK EE ES FI FR GB GR HU IE IS IT LI LT LU LV MC MT NL PL PT RO SE SI SK TR<br>Designated Extension States:<br>AL BA HR MK YU<br><br>(30) Priority: 20.06.2006 EP 06115736<br><br>(71) Applicant: Octapharma AG<br>8853 Lachen (CH) | (72) Inventors:<br>• Gruber, Gerhard<br>1210 Wien (AT)<br>• Reschny, Anton<br>1210 Wien (AT)<br><br>(74) Representative: Meyers, Hans-Wilhelm<br>Patentanwälte<br>von Kreisler-Selting-Werner<br>Postfach 10 22 41<br>50462 Köln (DE) |

(54) **Lyophilisation targeting defined residual moisture by limited desorption energy levels**

(57)    A process of freeze drying of an essentially aqueous solution comprising at least one first step having a first temperature and pressure level (i.e. primary drying phase) and at least one second step having a second temperature and pressure level (i.e. secondary drying phase) following the first step, wherein in the secondary drying phase limited desorption energy input is applied.

Figure 1

## Description

The invention pertains to a process of freeze drying of an essentially aqueous solution.

Background of the invention

Lyophilisation (freeze drying) is a stabilising process in which the formulation is first frozen, i.e., a separation of the solvent and the solutes, and then the concentration of the solvent, mainly water, is reduced first by sublimation (primary drying) and then by desorption (secondary drying) to levels that will no longer support biological growth or chemical reaction. [Lyophilization Seminar Notes, 1993, p.30]

Within the last decades freeze drying has become the most common method for the preparation of pharmaceuticals which are not adequately stable in solution. Among them the increasing demand for complex organic molecules, peptides and (recombinant) proteins being used as medicinal preparations have been the driving force to more scientifically investigate freeze drying cycles.

Being time consuming and expensive freeze drying is often the rate limiting process in the biopharmaceutical industries. Despite this disadvantage experience has shown that a variety of chemical reactions in aqueous solution, many of which are unacceptable in terms of product safety, are retarded in the dry state (e.g. hydrolysis, disulfide rearrangement, oxidation, aggregation etc.). Hence, freeze drying is considered one of the best methods to preserve proteins yielding products that are stable and convenient to store, ship or handle and which is accepted by the authorities as a suitable process step in the manufacture of therapeutic products.

Summary of the invention

As mentioned above, the secondary drying is typically related to desorption of water to target levels that will allow to use and/or store the formulation in a stable state. The secondary drying is usually performed at low chamber pressure and increased shelf temperatures for a distinct (limited) period of time until the target level of water (herein also referred to as residual moisture, RM) is achieved. However, by doing so several prerequisites must be fulfilled. For example, a homogenous temperature distribution of shelves and lyophilisator chamber (no hot/cold spots), a regular contact of vials between each other and between shelf surfaces leading to controlled heat transfer, all vials must be of identical quality (i.e. even thickness of glass wall and bottom), the product must have reached an almost identical physical state within each and every vial, etc. Taken together, since above mentioned prerequisites are hardly under control negative impact on the performance and results of a freeze drying cycle and the product quality thereof cannot be excluded.

In one embodiment of the invention the limited desorption energy input applied results in a desorption rate at the target rest moisture of the product approaching zero.

In another embodiment the process of the invention comprises a first shelf temperature level and a first chamber pressure level (i.e. primary or sublimation drying phase) and at least one second step having a second shelf temperature level and a second chamber pressure level (i.e. secondary or desorption drying phase) following the first step, characterized in that in the secondary or desoprtion drying phase at which the desorption rate at the target at rest moisture of the product approaches zero.

The "secondary drying (SD) phase" of a freeze drying cycle is per definition characterised by desorption i.e. removal of bound water. Although some other freeze drying parameters (e.g. chamber pressure, condenser temperature, etc.) applied during secondary drying must be considered the shelf temperature is the driving force for desorption (or the desorption rate if the amount of desorbed water in percent of solids per time [%/h] is calculated).

From the desorption rate the amount of desorbable water can be calculated at a given time point as follows:

$$\mathrm{D}Wt = {}_{t=0}\int^{t=1} \mathrm{D}R \, \mathrm{d}t$$

Desorbable water represents the amount of water still present in the product that can be removed by desorption. This must however not necessarily represent the identical amount of water that is determined by e.g. Karl Fischer titration or gravimetric methods for rest moisture determination since not all water present in a product can be removed by desorption.

By measuring the pressure increase during SD (i.e. close the valve between chamber and condenser for a defined period of time; so called "pressure rise measurement") the desorption rate and consequently the desorbable water $\mathrm{d}W$ can be estimated.

The $\mathrm{d}W$ and the corresponding rest moisture of a given substance must, however, be empirically estimated for every individual product due to above mentioned discrepancy between desorbable water and rest moisture determination. The desired $\mathrm{d}W$ value (corresponding to the desired rest moisture content) can afterwards be set as a process lead parameter at which predefined level the SD is terminated).

By keeping other freeze drying parameters constant (e.g. chamber pressure, condenser temperature) the desorption rate at a given shelf temperature decreases and approaches zero. In other words, even after extended SD time a certain, constant rest moisture content of the product will be approached. By determination of rest moisture levels after SD at different shelf temperatures a particular shelf temperature (or shelf temperature range) can empirically be defined where the corresponding rest moisture of the product reaches the targeted value and remains almost constant even in case of an extended SD phase.

This is in contrast to commonly applied freeze drying processes where the SD must be terminated or is automatically terminated by time.

Surprisingly it was found that the residual moisture could be targeted by adjusting the shelf temperature to levels even below temperature levels selected for primary drying. This method (i.e. the adjustment of the shelf temperature correlating with a limited level of desorption energy) allowed to perform the secondary step of a freeze drying cycle widely independent from duration of this particular step. As a consequence of this particular measure the process economy can be increased, e.g. by a reduction of rejections due to RM levels out of specification.

Figure 1 depicts a schematic drawing of a conventional and the freeze drying cycle of the invention.

Figure 2 shows an example of the invention.

Detailed description of the invention

The invention discloses a process of freeze drying of an essentially aqueous solution comprising at least one first step having a first temperature and pressure level (i.e. primary drying phase) and at least one second step having a second temperature and pressure level following the first step (i.e. secondary drying phase).

The secondary drying phase is characterized by desorption, which refers to the removal of physically bound water. The amount of desorbable water of a given substance is dependent on the level of energy input typically controlled by heat transfer e.g. via shelves. In conventional freeze drying cycles an excess of energy input is applied, hence the secondary drying phase has to be terminated by time. In contrast, the end point of the process of invention is reached when the desorption approaches zero, that is when under predefined conditions the desorbable water has been removed.

The process of the invention is advantageous since the residual moisture of the formulation can be adjusted to allow further treatment or dedicated use, e.g. thermal treatment for inactivation of chemical or biological compounds, shelf storage and other parameters.

The residual moisture of the formulation is homogenously distributed in the dried samples and prolongation of secondary drying phase does not lead to deleterious effects on the characteristics of the formulation (e.g. overdrying) or a residual moisture content out of specification.

Typically, according to the process of the invention biopolymers such as proteins, polysaccharides, nucleic acids, hormones can be dried. In particular, the biopolymers are pharmaceutical active substances derived from plasma or the corresponding active substances produced by recombinant technology, such as enzymes and their co-factors and inhibitors, hormones, immunoglobulins, growth factors or substances with multipotent function and activity. In particular the aqueous solution to be freeze-dried contains plasma proteins such as blood plasma, coagulation factors, proteases and cofactors, protease inhibitors, immunoglobulins, albumin and mixtures thereof. Furthermore, the proteins may be one ore more growth factors, hormones, cytokines and mixtures thereof. There is no difference in proteins derived from natural sources or recombinantly produced once or such which were produced transgenically.

When the substances were dissolved in a buffer system, the dried samples also contained buffer substances such as salts used in the biochemical field. If the freeze-dried proteins shall be used in the pharmaceutical field, it is advantageous to freeze-dry in presence of pharmaceutically acceptable salts.

In another embodiment of the invention there may be present salts or other ingredients which ease the later reconstitution of the freeze-dried proteins. Reconstitution may be improved when also detergents are present. Typically, the substances for improving the reconstitution are present in amounts of from 0.001 g/l to 10 g/l, in particular 0.01 g/l to 1.0 g/l.

For example, a human plasma derived protease or cofactor preparation belonging to the coagulation factor family, comprising of the biologically active compound in a buffered salt solution will be cooled to temperatures below 0°C, preferably below the onset of the melting point of the given product as a bulk preparation or in a solid containment like glass vials, ampoules or plastic containments, the latter consisting as a whole or partly of a vapour permeable membrane. To allow closure of the containment under vacuum or defined pressure of inert gases the vials are half closed with stoppers applicable for lyophilisation.

Having the containment in a freeze dryer (in principle consisting of a vacuum chamber with cooled/heated shelves, and a condenser chamber with cooled/heated condenser separated by a valve and the technical equipment to adjust/control temperatures and pressures of the equipment) the chamber pressure is reduced to levels lower than ambient pressure, preferably at $\leq 0.3$ mbar and the condenser is cooled to temperatures below that of the shelves. Subsequently, the temperature of shelves is increased to > 0°C, preferably to +20° to +30°C or higher. Depending of the filling volume or the amount of product the conditions of this phase, i.e. primary drying, are hold for > 6 hours or at least until the temperature of the product measured by temperature probes will approximate shelf temperature thus indicating the end of primary drying phase.

Then the secondary drying phase is initiated by lowering the chamber pressure to 0.1 mbar or less, for example 0.01 mbar or less. In contrast to the most commonly applied parallel increase of shelf temperature a set point of the shelf temperature to levels even below those during primary drying is targeted. After ≥ 4 hours secondary drying phase the freeze drying process is terminated by closing the vials, either at vacuum level as defined for secondary drying or under defined pressure using inert gas, e.g. nitrogen.

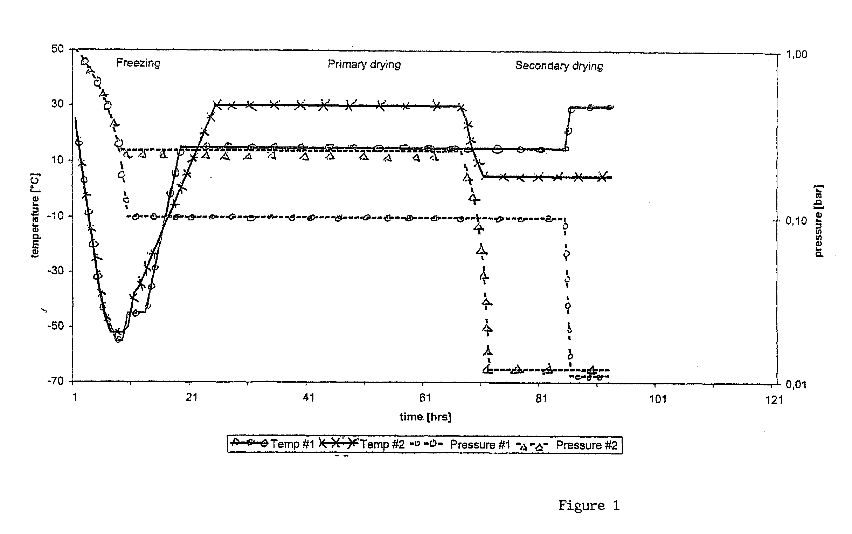

Figure 1 depicts a schematic drawing of a typical conventional freeze drying cycle (Temperature #1, Pressure #1). In contrast to the shelf temperature increase during secondary drying a decrease of shelf temperature according to the invention is applied (Temperature #2, see arrow) allowing to target the RM of a freeze dried substance. The pressure course of the method of the invention is shown as Pressure #2.

According to the invention more homogeneous residual moisture (RM) distribution can be achieved.

The invention is further explained by way of the non-limiting example.

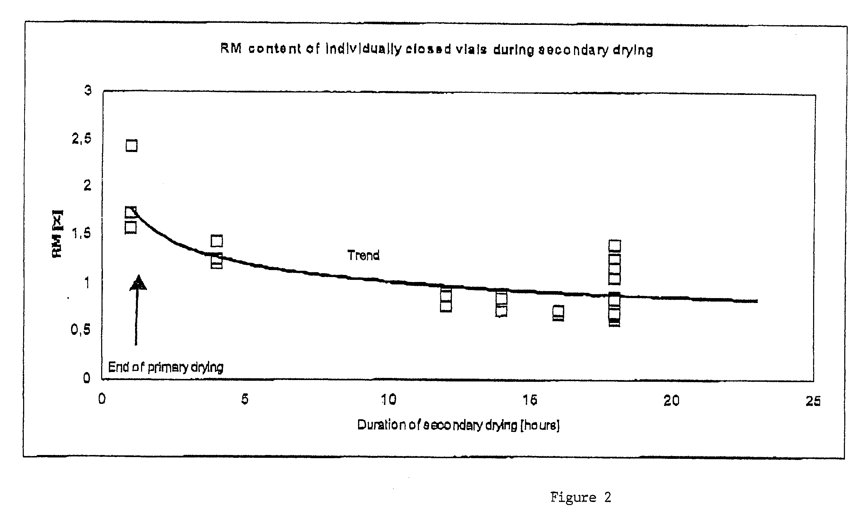

A human plasma derived protein component was subjected to freeze drying. After switching from the main drying to the secondary drying phase vials (n=3) were individually closed (by a sample manipulator) at predefined time points during secondary drying. At the end of the FD experiment the remaining vials were closed and subsequently all vials were subjected to rest moisture determination. In Figure 2 the rest moisture values were plotted against time (duration of secondary drying) and the trend line was calculated.

It is obvious that at a given shelf temperature the rest moisture of the product reaches a constant level independent on the duration of the secondary drying phase.

In additional experiments using another human plasma derived product the influence of the shelf temperature on the rest moisture content of a given product is listed in the table below. The parameters time and chamber pressure during secondary drying were kept constant while only the shelf temperature was varied. The corresponding rest moisture values of each experiment are also listed. It is obvious that with increasing shelf temperature the mean rest moisture of the product is decreasing. Thus, the control (or limitation) of desorption energy input which is technically equivalent to shelf temperature is an effective tool to adjust a target rest moisture of a given product widely independent of secondary drying time.

| Secondary Drying | Mean reast moisture |
|---|---|
| 3h at -5°C, 0.01mbar | 1.14 |
| 3h at -2°C, 0.01mbar | 0.92 |
| 3h at -2°C, 0.01mbar | 0.98 |
| 3h at -2°C, 0.01mbar | 0.86 |
| 3h at -2°C, 0.01mbar | 0.93 |
| 3h at -2°C, 0.01mbar | 1.03 |
| 3h at -2°C, 0.01mbar | 0.94 |

(continued)

| Secondary Drying | Mean reast moisture |
|---|---|
| Mean value (n=6) | 0.96 |
| 3h at +3°C, 0.01mbar | 0.79 |
| 3h at +3°C, 0.01mbar | 0.85 |
| Mean value (n=2) | 0.82 |

**Claims**

1. A process of freeze drying of an essentially aqueous solution comprising at least one first step having a first temperature and pressure level (i.e. primary drying phase) and at least one second step having a second temperature and pressure level (i.e. secondary drying phase) following the first step, wherein in the secondary drying phase limited desorption energy input is applied.

2. The process of claim 1 wherein the limited desorption energy input applied results in a desorption rate at the target rest moisture of the product approaching zero.

3. The process of claim 1 and/or 2 having a first shelf temperature level and a first chamber pressure level (i.e. primary or sublimation drying phase) and at least one second step having a second shelf temperature level and a second chamber pressure level (i.e. secondary or desorption drying phase) following the first step, **characterized in that** in the secondary or desoprtion drying phase at which the desorption rate at the target at rest moisture of the product approaches zero.

4. The process of at least one of the claims 1 to 3 wherein the aqueous solution contains biopolymers such as proteins, polysaccharides, nucleic acids.

5. The process of at least one of the claims 1 to 4, wherein the biopolymers are pharmaceutically active substances.

6. The process of any one of the claims 1 to 5, wherein the aqueous solution contains proteins selected from the group consisting of plasma proteins such as blood plasma, coagulation factors, proteases or co-factors, protease inhibitors, immunoglobulins, albumin and mixtures thereof.

7. The process of any one of the claims 1 to 6, wherein the aqueous solution contains proteins selected from the group consisting of one or more growth factors, hormones, cytokines and mixtures thereof.

**8.** The process of any one of the claims 1 to 7, wherein the aqueous solution contains recombinantly or transgenically produced proteins.

**9.** The process of any one of the claims 1 to 8, wherein the aqueous solution contains prior to drying additionally buffer substances.

Figure 1

Figure 2

**European Patent Office**

**EUROPEAN SEARCH REPORT**

Application Number

EP 07 11 0675

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/043042 A1 (JOHNSON AUDREY M [US] ET AL) 4 March 2004 (2004-03-04) * paragraphs [0025], [0026], [0034], [0035], [0040] *<br>----- | 1-9 | INV.<br>F26B5/06<br>A61K9/19 |
| A | ADAMS G D J: "FREEZE-DRYING OF BIOLOGICAL MATERIALS"<br>DRYING TECHNOLOGY, TAYLOR & FRANCIS, PHILADELPHIA, PA, US,<br>vol. 9, no. 4,<br>1 September 1991 (1991-09-01), pages 891-925, XP000232604<br>ISSN: 0737-3937<br>----- | | |
| A | WO 03/026786 A2 (CLEARANT INC [US]; BURGESS WILSON H [US]; DROHAN WILLIAM N [US]; MACPH) 3 April 2003 (2003-04-03)<br>----- | | |
| A | US 2005/226893 A1 (JUNEAU JENNIFER [US] ET AL) 13 October 2005 (2005-10-13)<br>----- | | |

**TECHNICAL FIELDS SEARCHED (IPC)**

F26B
A61K

The present search report has been drawn up for all claims

| Place of search | Date of completion of the search | Examiner |
|---|---|---|
| The Hague | 11 October 2007 | Silvis, Henk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 11 0675

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2007

| Patent document cited in search report | | Publication date | Patent family member(s) | | Publication date |
|---|---|---|---|---|---|
| US 2004043042 | A1 | 04-03-2004 | NONE | | |
| WO 03026786 | A2 | 03-04-2003 | AU | 2002326819 A1 | 07-04-2003 |
| | | | US | 2003068416 A1 | 10-04-2003 |
| US 2005226893 | A1 | 13-10-2005 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Lyophilization Seminar Notes,* 1993, 30